# EUROPEAN PATENT APPLICATION

(11) **EP 3 831 442 A1**
(43) Date of publication of application: **09.06.2021**
(21) Application number: 20211213.2
(22) Date of filing: 02.12.2020
(51) Int. Cl.: A61N 1/32, A61B 18/12, A61B 18/14, A61B 90/00, A61B 5/01, A61B 5/05, A61B 18/00

(54) **USING REVERSIBLE ELECTROPORATION ON CARDIAC TISSUE**

(30) Priority: 03.12.2019 US 201962942999 P; 06.07.2020 US 202016921578
(71) Applicant: Biosense Webster (Israel) Ltd, Yokneam, 2066717 (IL)
(72) Inventor: ALTMANN, Andres Claudio, 2066717 Yokneam (IL); GOVARI, Assaf, 2066717 Yokneam (IL)
(74) Representative: Small, Gary James

(57) **Abstract**

In one embodiment, an electroporation method includes inserting a catheter having multiple electrodes into a chamber of a heart, applying an electrical field using at least two of the electrodes to tissue of the chamber of the heart at a given location within the chamber with an amplitude sufficient to cause reversible electroporation, but below a threshold for irreversible electroporation, and measuring an effect of the reversible electroporation on electrical activation signals in the tissue of the chamber of the heart in a vicinity of the location.

## Description

### RELATED APPLICATION INFORMATION

The present application claims benefit of US Provisional Patent Application Serial Number 62/942,999 to Altmann, et al., filed 3 December 2019, the disclosure of which is hereby incorporated herein by reference.

### FIELD OF THE INVENTION

The present invention relates to medical systems, and in particular, but not exclusively, to reversible electroporation.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/006455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied to the tip electrode(s) of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

US Patent Publication No. 2017/0348525 to Sano, et al., describes a ratio of reversible electroporation and irreversible electroporation controlled by selecting a symmetric waveform or asymmetric waveform to either minimize or enhance irreversible effects on cells in the target tissue. Combined reversible and irreversible electroporation includes inserting one or more therapeutic electrodes into a target tissue, introducing an electroporation compound into the target tissue, selecting a pulse waveform that is either 1) asymmetric bipolar that has positive and negative pulses with different durations, or 2) symmetric bipolar that has positive and negative pulses with the same duration, and delivering to the target tissue a series of electrical pulses having the selected pulse waveform.

US Patent No. 10,245,098 to Davalos, et al., describes a method for ablating brain tissue of a living mammal comprising: placing first and second electrodes in a brain of the living mammal; applying a plurality of electrical pulses through the first and second placed electrodes which are predetermined to: cause irreversible electroporation (IRE) of brain tissue of the mammal within a target ablation zone; and cause a temporary disruption of a blood brain barrier (BBB) within a surrounding zone that surrounds the target ablation zone to allow material in a blood vessel to be transferred to the surrounding zone through the temporarily disrupted BBB. Such methods are useful for delivering large molecule material within a blood vessel of the brain across the BBB, where the large molecule is otherwise blocked by the BBB from passing through the blood vessel into the brain.

US Patent No. 10,154,874 to Davalos, et al., describes methods for treating tissue with irreversible electroporation and immunotherapy. The methods include placing a probe in tissue within a human body, wherein the probe has at least a first electrode, applying a plurality of electrical pulses through the first electrode and a second electrode, causing irreversible electroporation (IRE) of the tissue within a target ablation zone, and administering one or more exogenous agents into the tissue within the target ablation zone or to the human, thereby stimulating or otherwise modulating an immune system response within the body.

US Patent No. 10,010,666 to Rubinsky, et al., provides a balloon catheter with a particular electrode configuration. Also provided is a method whereby vascular cells in the area of the artery subjected to the trauma are subjected to irreversible electroporation which is a non-thermal, non-pharmaceutical method of applying electrical pulses to the cells so that substantially all of the cells in the area are ablated while leaving the structure of the vessel in place and substantially unharmed due to the non-thermal nature of the procedure.

US Patent No. 9,345,538 to Deem, et al., describes methods and apparatus for selective destruction or temporary disruption of nerves and/or conduction pathways in a mammalian body for the treatment of pain and other disorders. Apparatus comprises catheters having electrodes for targeting and affecting nerve tissue at a cellular level to reversible and irreversible nerve poration and incapacitation.

US Patent No. 7,937,143 to Demarais, et al., describes methods and apparatus for inducing, monitoring and controlling renal neuromodulation using a pulsed electric field to effectuate electroporation or electrofusion. In some embodiments, tissue impedance, conductance or conductivity may be monitored to determine the effects of pulsed electric field therapy, e.g., to determine an extent of electroporation and its degree of irreversibility. Pulsed electric field electroporation of tissue causes a decrease in tissue impedance and an increase in tissue conductivity. If induced electroporation is reversible, upon cessation of the pulsed electric field, tissue impedance and conductivity should approximate baseline levels; however, if electroporation is irreversible, impedance and conductivity changes should persist. Thus, monitoring of impedance or conductivity may be utilized to determine the onset of electroporation and to determine the type or extent of electroporation. Furthermore, monitoring data may be used in one or more manual or automatic feedback loops to control the electroporation.

### SUMMARY

There is provided in accordance with an embodiment of the present invention, a electroporation method, including inserting a catheter having multiple electrodes into a chamber of a heart, applying an electrical field using at least two of the electrodes to tissue of the chamber of the heart at a given location within the chamber with an amplitude sufficient to cause reversible electroporation, but below a threshold for irreversible electroporation, and measuring an effect of the reversible electroporation on electrical activation signals in the tissue of the chamber of the heart in a vicinity of the location.

Further in accordance with an embodiment of the present invention the electrical field is less than 450 Volts per centimeter.

Still further in accordance with an embodiment of the present invention, the method includes generating a pulsed electrical signal and wherein the applying the electrical field includes applying the electrical field using the at least two electrodes responsively to the generated pulsed electrical signal.

Additionally, in accordance with an embodiment of the present invention the pulsed electrical signal includes a series of biphasic pulses, each biphasic pulse including a positive and a negative phase pulse.

Moreover, in accordance with an embodiment of the present invention the pulsed electrical signal includes a series of bursts, each burst including a series of pulses.

Further in accordance with an embodiment of the present inention each of the pulses has a pulse length between 1 and 20 microseconds, and the series of bursts includes a gap between bursts of between 100 microseconds to 1000 milliseconds.

Still further in accordance with an embodiment of the present invention each burst includes up to 100 of the pulses, and the series of bursts includes up to 100 bursts.

Additionally in accordance with an embodiment of the present invention, the method includes rendering to a display an indication of the electrical activation signals in the tissue of the chamber of the heart in the vicinity of the location, and then applying another electrical field using at least two of the electrodes to the tissue of the chamber of the heart at the given location within the chamber with an amplitude sufficient to cause irreversible electroporation.

Moreover, in accordance with an embodiment of the present invention, the method includes generating an electroanatomic map of the chamber of the heart responsively to the electrical activation signals, and rendering the electroanatomic map to the display.

Further in accordance with an embodiment of the present invention the electric field with the amplitude sufficient to cause reversible electroporation but below a threshold for irreversible electroporation is less than 450 Volts per centimeter, and the other electric field with the amplitude sufficient to cause irreversible electroporation is greater than 800 Volts per centimeter.

Still further in accordance with an embodiment of the present invention, the method includes generating a pulsed electrical signal and wherein the applying the other electrical field includes applying the other electrical field using the at least two electrodes responsively to the generated pulsed electrical signal.

Additionally, in accordance with an embodiment of the present invention the pulsed electrical signal includes a series of biphasic pulses, each biphasic pulse including a positive and a negative phase pulse.

Moreover, in accordance with an embodiment of the present invention the pulsed electrical signal includes a series of bursts, each burst including a series of pulses.

Further in accordance with an embodiment of the present invention each of the pulses has a pulse length between 1 and 20 microseconds, and the series of bursts includes a gap between bursts of between 100 microseconds to 1000 milliseconds.

Still further in accordance with an embodiment of the present invention each burst includes up to 100 of the pulses, and the series of bursts includes up to 100 bursts.

There is also provided in accordance with another embodiment of the present invention a electroporation system, including a catheter including multiple electrodes, and configured to be inserted into a chamber of a heart, a signal generator coupled to at least two of the electrodes, and configured to generate an electrical signal for supply to the at least two electrodes which responsively to the electrical signal apply an electrical field to tissue of the chamber of the heart at a given location within the chamber, the electrical field having an amplitude sufficient to cause reversible electroporation, but below a threshold for irreversible electroporation, and processing circuitry configured to receive from the catheter electrical activation signals in the tissue of the chamber of the heart in a vicinity of the location, and measure an effect of the reversible electroporation on the electrical activation signals in the tissue of the chamber of the heart in a vicinity of the location.

Additionally, in accordance with an embodiment of the present invention the electrical field is less than 450 Volts per centimeter.

Moreover, in accordance with an embodiment of the present invention the electrical signal is a pulsed electrical signal.

Further in accordance with an embodiment of the present invention the pulsed electrical signal includes a series of biphasic pulses, each biphasic pulse including a positive and a negative phase pulse.

Still further in accordance with an embodiment of the present invention the pulsed electrical signal includes a series of bursts, each burst including a series of pulses.

Additionally, in accordance with an embodiment of the present invention each of the pulses has a pulse length between 1 and 20 microseconds, and the series of bursts includes a gap between bursts of between 100 microseconds to 1000 milliseconds.

Moreover, in accordance with an embodiment of the present invention each burst includes up to 100 of the pulses, and the series of bursts includes up to 100 bursts.

Further in accordance with an embodiment of the present invention the processing circuitry is configured to render to a display an indication of the electrical activation signals in the tissue of the chamber of the heart in the vicinity of the location, and the signal generator is configured to generate another electrical signal for supply to at least two of the electrodes which responsively to the other electrical signal apply another electrical field to tissue of the chamber of the heart at the location within the chamber with an amplitude sufficient to cause irreversible electroporation.

Still further in accordance with an embodiment of the present invention the processing circuitry is configured to generate an electroanatomic map of the chamber of the heart responsively to the electrical activation signals, and render the electroanatomic map to the display.

Additionally, in accordance with an embodiment of the present invention the electric field with the amplitude sufficient to cause reversible electroporation but below a threshold for irreversible electroporation is less than 450 Volts per centimeter, and the other electric field with the amplitude sufficient to cause irreversible electroporation is greater than 800 Volts per centimeter.

Moreover, in accordance with an embodiment of the present invention the other electrical signal is a pulsed electrical signal.

Further in accordance with an embodiment of the present invntion the pulsed electrical signal includes a series of biphasic pulses, each biphasic pulse including a positive and a negative phase pulse.

Still further in accordance with an embodiment of the present invention the pulsed electrical signal includes a series of bursts, each burst including a series of pulses.

Additionally, in accordance with an embodiment of the present invention each of the pulses has a pulse length between 1 and 20 microseconds, and the series of bursts includes a gap between bursts of between 100 microseconds to 1000 milliseconds.

Moreover, in accordance with an embodiment of the present invention each burst includes up to 100 of the pulses, and the series of bursts includes up to 100 bursts.

### BRIEF DESCRIPTION OF THE DRAWINGS

The present invention will be understood from the following detailed description, taken in conjunction with the drawings in which:
Fig. 1 is a schematic view of a medical procedure system constructed and operative in accordance with an exemplary embodiment of the present invention;
Fig. 2 is a schematic view of a catheter for use in the system of Fig. 1;
Fig. 3 is a flowchart including steps in a method of operation of the medical procedure system of Fig. 1;
Fig. 4 is a schematic view of intracardiac electrograms generated by the system of Fig. 1; and
Fig. 5 is a schematic view of an electroanatomic map generated by the system of Fig. 1.

### DESCRIPTION OF EXAMPLE EMBODIMENTS

### OVERVIEW

Irreversible electroporation (IRE) applies short electrical pulses that generate high enough electrical fields (typically greater than 450 Volts per centimeter) to irreversibly damage the cells. Non-thermal IRE may be used in treating different types of tumors and other unwanted tissue without causing thermal damage to surrounding tissue. Small electrodes are placed in proximity to target tissue to apply short electrical pulses. The pulses increase the resting transmembrane potential, so that nanopores form in the plasma membrane. When the electricity applied to the tissue is above the electric field threshold of the target tissue, the cells become permanently permeable from the formation of nanopores. As a result, the cells are unable to repair the damage and die due to a loss of homeostasis and the cells typically die by apoptosis.

IRE may be used for cardiac ablation as an alternative to other cardiac ablation techniques, e.g., radio-frequency (RF) cardiac ablation. IRE cardiac ablation is sometimes referred to as Pulse Field Ablation (PFA). As IRE is generally a low thermal technique, IRE may reduce the risk of collateral cell damage that is present with the other techniques. e.g., in RF cardiac ablation. However, similar to other ablation techniques, such as RF cardiac ablation, until after IRE pulses have actually been applied to a target location of the heart, thereby killing the cells in the target location, a physician may not be certain that killing the cells of the target location is the correct procedure to be followed.

Exemplary embodiments of the invention solve the above problems by applying an electrical field to induce reversible electroporation at a target location. Reversible electroporation applies short electrical pulses, generating high electrical fields (up to around 450 Volts per centimeter), to cells so as to temporarily break down the membranes of cells. Reversible electroporation occurs when the electricity applied with the electrodes is below the electric field threshold of the target tissue allowing cells to repair. Reversible electroporation does not kill the cells, but allows a physician to see the effect of reversible electroporation on electrical activation signals in the vicinity of the target location.

While the reversible electroporation is being induced, or substantially immediately afterwards, for example, up to about a few seconds afterwards. electrical activation signals from the vicinity of the target location are acquired. The acquired signals may be processed to provide a visual indication or indications of the electrical activation signals. The visual indication(s) may be compared with those prior to the application of the reversible electroporation, to check if an expected change in the electrical activity of the target location has actually occurred. Additionally, or alternatively, the electrical activation signals may be processed to generate an electroanatomic map which may then be compared with an electroanatomic map generated prior to the application of the reversible electroporation.

After the effect of the reversible electroporation on electrical activation signals in the vicinity of the target location has been evaluated by the physician, the physician may decide whether or not to perform another procedure, such as, IRE or RF cardiac ablation at the target location. For example, if an expected change has in fact occurred, IRE (or another suitable ablation technique) may be applied to the target location to irreversibly kill the cells of the target location. If no expected change is observed, IRE (or another suitable ablation technique) may not be applied to the location.

Applying reversible electroporation to a target location prior to applying IRE (or another suitable ablation technique) to the location allows the physician to check if ablating the location gives an expected result. The reversibility of reversible electroporation enables the physician to perform the check without there being any permanent damage to the heart tissue.

### SYSTEM DESCRIPTION

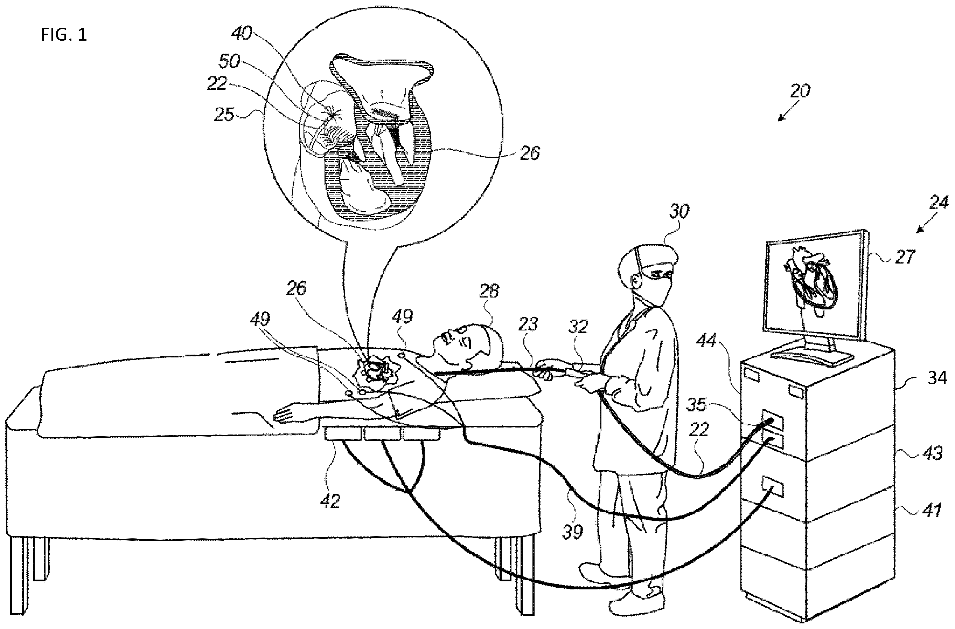

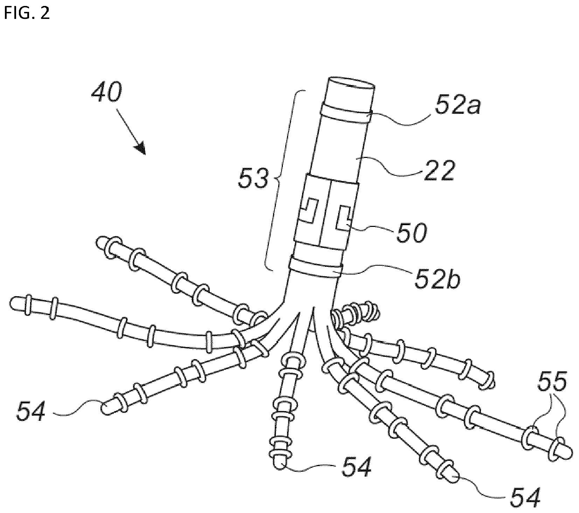

Reference is now made to Fig. 1, which is a schematic view of a medical procedure system 20 constructed and operative in accordance with an embodiment of the present invention. Reference is also made to Fig. 2, which is a schematic view of a catheter 40 for use in the system 20 of Fig. 1.

The medical procedure system 20 is used to determine the position of the catheter 40, seen in an inset 25 of Fig. 1 and in more detail in Fig. 2. The catheter 40 includes a shaft 22 and a plurality of deflectable arms 54 (only some labeled for the sake of simplicity) for inserting into a body-part of a living subject. The deflectable arms 54 have respective proximal ends connected to the distal end of the shaft 22.

The catheter 40 includes a position sensor 53 disposed on the shaft 22 in a predefined spatial relation to the proximal ends of the deflectable arms 54. The position sensor 53 may include a magnetic sensor 50 and/or at least one shaft electrode 52. The magnetic sensor 50 may include at least one coil, for example, but not limited to, a dual-axis or a triple axis coil arrangement to provide position data for location and orientation including roll. The catheter 40 includes multiple electrodes 55 (only some are labeled in Fig. 2 for the sake of simplicity) disposed at different, respective locations along each of the deflectable arms 54. Typically, the catheter 40 may be used for mapping electrical activity in a heart of the living subject using the electrodes 55, or for performing any other suitable function in a body-part of a living subject, for example, but not limited to, reversible and/or irreversible electroporation and/or RF ablation.

The medical procedure system 20 may determine a position and orientation of the shaft 22 of the catheter 40 based on signals provided by the magnetic sensor 50 and/or the shaft electrodes 52 (proximal-electrode 52a and distal-electrode 52b) fitted on the shaft 22, on either side of the magnetic sensor 50. The proximal-electrode 52a, the distal-electrode 52b, the magnetic sensor 50 and at least some of the electrodes 55 are connected by wires running through the shaft 22 via a catheter connector 35 to various driver circuitries in a console 24. In some embodiments, at least two of the electrodes 55 of each of the deflectable arms 54, the shaft electrodes 52, and the magnetic sensor 50 are connected to the driver circuitries in the console 24 via the catheter connector 35. In some embodiments, the distal-electrode 52b and/or the proximal electrode 52a may be omitted.

The illustration shown in Fig. 2 is chosen purely for the sake of conceptual clarity. Other configurations of shaft electrodes 52 and electrodes 55 are possible. Additional functionalities may be included in the position sensor 53. Elements which are not relevant to the disclosed embodiments of the invention, such as irrigation ports, are omitted for the sake of clarity.

A physician 30 navigates the catheter 40 to a target location in a body part (e.g., a heart 26) of a patient 28 by manipulating the shaft 22 using a manipulator 32 near the proximal end of the catheter 40 and/or deflection from a sheath 23. The catheter 40 is inserted through the sheath 23, with the deflectable arms 54 gathered together, and only after the catheter 40 is retracted from the sheath 23, the deflectable arms 54 are able to spread and regain their intended functional shape. By containing deflectable arms 54 together, the sheath 23 also serves to minimize vascular trauma on its way to the target location.

Console 24 comprises processing circuitry 41, typically a general-purpose computer and a suitable front end and interface circuits 44 for generating signals in, and/or receiving signals from, body surface electrodes 49 which are attached by wires running through a cable 39 to the chest and to the back, or any other suitable skin surface, of the patient 28.

Console 24 further comprises a magnetic-sensing sub-system. The patient 28 is placed in a magnetic field generated by a pad containing at least one magnetic field radiator 42, which is driven by a unit 43 disposed in the console 24. The magnetic field radiator(s) 42 is configured to transmit alternating magnetic fields into a region where the body-part (e.g., the heart 26) is located. The magnetic fields generated by the magnetic field radiator(s) 42 generate direction signals in the magnetic sensor 50. The magnetic sensor 50 is configured to detect at least part of the transmitted alternating magnetic fields and provide the direction signals as corresponding electrical inputs to the processing circuitry 41.

In some embodiments, the processing circuitry 41 uses the position-signals received from the shaft electrodes 52, the magnetic sensor 50 and the electrodes 55 to estimate a position of the catheter 40 inside an organ, such as inside a cardiac chamber. In some embodiments, the processing circuitry 41 correlates the position signals received from the electrodes 52, 55 with previously acquired magnetic location-calibrated position signals, to estimate the position of the catheter 40 inside a cardiac chamber. The position coordinates of the shaft electrodes 52 and the electrodes 55 may be determined by the processing circuitry 41 based on, among other inputs, measured impedances, or on proportions of currents distribution, between the electrodes 52, 55 and the body surface electrodes 49. The console 24 drives a display 27, which shows the distal end of the catheter 40 inside the heart 26.

The method of position sensing using current distribution measurements and/or external magnetic fields is implemented in various medical applications, for example, in the Carto® system, produced by Biosense Webster Inc. (Irvine, California), and is described in detail in U.S. Patent Nos. 5,391,199, 6,690,963, 6,484,118, 6,239,724, 6,618,612, 6,332,089, 7,756,576, 7,869,865, and 7,848,787, in PCT Patent Publication WO 96/05768, and in U.S. Patent Application Publication Nos. 2002/0065455 A1, 2003/0120150 A1 and 2004/0068178 A1.

The Carto®3 system applies an Active Current Location (ACL) impedance-based position-tracking method. In some embodiments, using the ACL method, the processing circuitry 41 is configured to create a mapping (e.g., current-position matrix (CPM)) between indications of electrical impedance and positions in a magnetic coordinate frame of the magnetic field radiator(s) 42. The processing circuitry 41 estimates the positions of the shaft electrodes 52 and the electrodes 55 by performing a lookup in the CPM.

Other methods of determining the location of the distal end of the catheter may be used, for example, based on ultrasonic transducers and receivers, using imaging techniques such as ultrasound or MRI or CT scans which may include disposing radiopaque tags on the catheter 40.

Processing circuitry 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

Fig. 1 shows only elements related to the disclosed techniques, for the sake of simplicity and clarity. The system 20 typically comprises additional modules and elements that are not directly related to the disclosed techniques, and thus are intentionally omitted from Fig. 1 and from the corresponding description.

The catheter 40 described above includes eight deflectable arms 54 with six electrodes per arm 54. Any suitable catheter may be used instead of the catheter 40, for example, a catheter with a different number of flexible arms and/or electrodes per arm, or a different probe shape such as a balloon catheter or a lasso catheter, by way of example only.

The medical procedure system 20 may also perform electroporation or RF ablation (or other ablation technique) of heart tissue using any suitable catheter, for example using the catheter 40 or a different catheter and any suitable ablation method. The console 24 may include a signal generator 34 configured to generate an electrical signal to be applied by an electrode or electrodes of a catheter connected to the console 24, (and optionally one or more of the body surface electrodes 49), to perform electroporation of RF ablation of a myocardium of the heart 26. The console 24 may include a pump (not shown), which pumps irrigation fluid into an irrigation channel to a distal end of a catheter performing RF ablation. The catheter performing the RF ablation may also include temperature sensors (not shown) which are used to measure a temperature of the myocardium during RF ablation and regulate an ablation power and/or an irrigation rate of the pumping of the irrigation fluid according to the measured temperature.

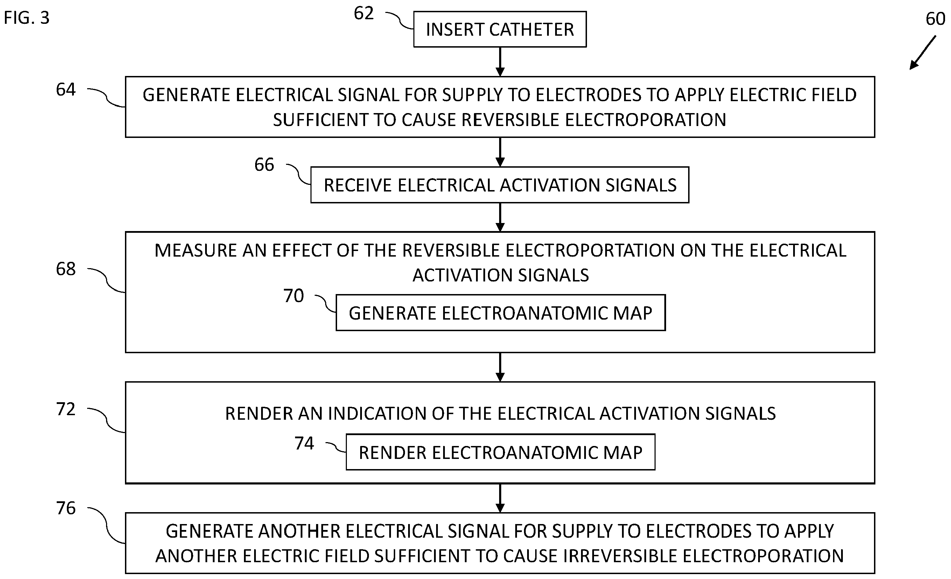

Reference is now made to Fig. 3, which is a flowchart 60 including steps in a method of operation of the medical procedure system 20 of Fig. 1.

The catheter 40 (Fig. 1) is configured to be inserted (block 62) into a chamber of the heart 26 (Fig. 1).

The signal generator 34 (Fig. 1) is coupled to at least two of the electrodes 55 (Fig. 2) of the catheter 40. The signal generator 34 is configured to generate (block 64) an electrical signal for supply to at least two of the electrodes 55 which responsively to the electrical signal apply an electrical field to tissue of the chamber of the heart 26 at a given location within the chamber. The electrical field has an amplitude sufficient to cause reversible electroporation, but below a threshold for irreversible electroporation. The field may be applied between any two of the electrodes 55, for example, between adjacent electrodes 55, and/or between some of the electrodes 55 and a reference electrode of the electrodes 55. The electrical field applied by the electrodes 55 to the tissue at the given location is generally less than 450 Volts per centimeter. The electrical signal supplied by the signal generator 34 may be a pulsed electrical signal. The pulsed electrical signal may include a series of biphasic pulses, with each biphasic pulse including a positive and a negative phase pulse. For example, the series of biphasic pulses may include a positive phase pulse of 2 microseconds, followed by a delay of 0.5 microseconds, followed by a negative phase pulse of 2 microseconds, and so on. The pulsed electrical signal may include a series of bursts, with each burst including a series of pulses, such as the biphasic pulses or a single-phase pulse. Each of the pulses may have any suitable length, for example, between 1 and 20 microseconds. The series of bursts includes a gap between bursts of any suitable length, for example, between 100 microseconds to 1000 milliseconds. Each burst may include any suitable number of pulses, for example, up to 100 pulses. The series of bursts may include any suitable number of bursts, for example, up to 100 bursts.

The various parameters of the electrical signals (such as amplitude, pulse length, gap, and number of pulses and bursts) may be suitably adjusted to provide an electrical field which causes reversible electroporation into the tissue to a depth of at least 2-3 mm without substantially heating surrounding tissue. The term "without substantially heating surrounding tissue" as used in the specification and claims, is defined as heating the tissue to a level at which there is no clinical effect. In some applications a three degrees centigrade increase in temperature may be acceptable for the durations used in electroporation.

During the time period in which the electric field is being applied, or substantially immediately afterwards, the effect of the reversible electroporation on the electrical activation signals in the tissue of the chamber of the heart 26 in the vicinity of the location where the electric field was/is being applied is measured.

The processing circuitry 41 is configured to receive (block 66) from the catheter 40 electrical activation signals in the tissue of the chamber of the heart 26 in the vicinity of the location.

The processing circuitry 41 is configured to measure (block 68) an effect of the reversible electroporation on the electrical activation signals in the tissue of the chamber of the heart 26 in the vicinity of the location. Measuring an effect of the reversible electroporation may include measuring an amplitude of signals captured from the vicinity of the location, and/or processing the signals to generate graphs of the signals and/or generating an electroanatomic map based on the electrical activation signals. The "vicinity" of the location may be in and/or around the location at which the reversible electroporation is performed.

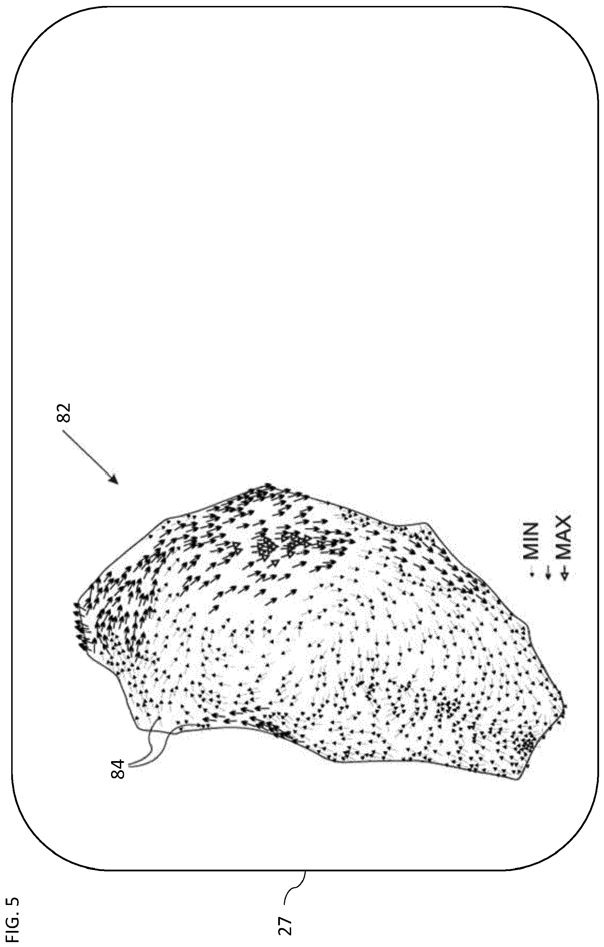

Therefore, in some embodiments, the processing circuitry 41 is configured to generate (block 70) an electroanatomic map of the chamber of the heart responsively to the electrical activation signals. The step of block 70 is described in more detail with reference to Fig. 5 below.

The processing circuitry 41 is configured to render (block 72) to the display 27 an indication of the electrical activation signals in the tissue of the chamber of the heart in the vicinity of the location. The indication may include an amplitude or amplitudes of the activation signals, a graph or graphs of the activation signals or an electroanatomic map representing the activation signals.

In some embodiments, before, during or after inducing the reversible electroporation in the tissue, a substance may be applied to the tissue, and the effect of the reversible electroporation and the substance on the electrical activation signals may be examined.

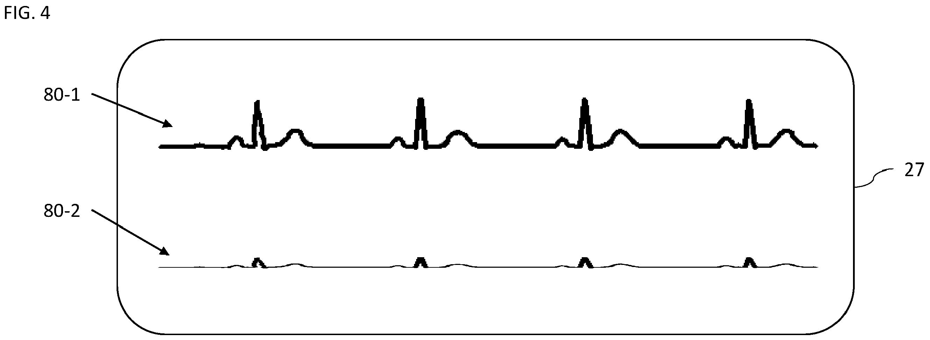

Reference is now made to Fig. 4, which is a schematic view of intracardiac electrograms 80 generated by the system 20 of Fig. 1. The intracardiac electrograms 80-1, 80-2 are rendered to the display 27 by the processing circuitry 41 (Fig. 1) and represent electrical activation signals captured at a point around the location of the reversible electroporation, prior to the reversible electroporation being applied (shown in intracardiac electrogram 80-1) and during or substantially immediately after the reversible electroporation has been applied (shown in intracardiac electrograms 80-2), respectively. It can be seen that the reversible electroporation has had an effect on reducing electrical activity at the point around the location.

Reference is now made to Fig. 5, which is a schematic view of an electroanatomic map 82 generated by the system 20 of Fig. 1. Reference is also made to Fig. 3.

The processing circuitry 41 (Fig. 1) is configured to process the electrical activation signals captured by the catheter 40 (Fig. 1) over time at multiple sample locations over the surface of the chamber(s) of the heart 26 to determine respective activation times at the multiple locations over the surface of the chamber(s) of the heart 26. The processing circuitry 41 is configured to prepare the electroanatomic map 82 including a plurality of velocity vectors 84 (only some labeled for the sake of simplicity) describing the propagation of activation wavefronts associated with the activation times. One method for preparing the electroanatomic map 82 is described in US Patent No. 6,301,496. Any suitable method for preparing the electroanatomic map 82 may also be used. Other electroanatomic maps may be generated for example, showing activation times using colors.

The processing circuitry 41 is configured to render (block 74) the electroanatomic map 82 to the display 27. The electroanatomic map 82 may be displayed alongside another electroanatomic map representing electrical activity of the chamber(s) of the heart 26 prior to the reversible electroporation being performed in order to further visualize the effect of the reversible electroporation.

Reference is again made to Fig. 3. The physician examines the intracardiac electrograms 80 (Fig. 4) and/or the electroanatomic map 82 (Fig. 5) or other data to determine if the location at which reversible electroporation was applied should be permanently ablated using IRE or another ablation technique or not ablated at all. If the physician decides to permanently ablate, the physician operates the system 20 to perform the ablation. The signal generator 34 (Fig. 1) is configured to generate another electrical signal for supply to at least two of the electrodes 55 which responsively to the other electrical signal apply another electrical field to tissue of the chamber of the heart 26 (Fig. 1) at the location within the chamber with an amplitude sufficient to cause irreversible electroporation (block 76). Alternatively, the physician could use another form of ablation, such as RF ablation which kills the cells of the tissue due to its heating effect.

The field may be applied between any two of the electrodes 55, for example, between adjacent electrodes 55, and/or between some of the electrodes 55 and a reference electrode of the electrodes 55.The electrical field applied by the electrodes 55 to the tissue at the given location is generally more than 800 Volts per centimeter. The electrical signal supplied by the signal generator 34 may be a pulsed electrical signal. The pulsed electrical signal may include a series of biphasic pulses, with each biphasic pulse including a positive and a negative phase pulse. For example, the series of biphasic pulses may include a positive phase pulse of 2 microseconds, followed by a delay of 0.5 microseconds, followed by a negative phase pulse of 2 microseconds, and so on. The pulsed electrical signal may include a series of bursts, with each burst including a series of pulses, such as the biphasic pulses or a single-phase pulse. Each of the pulses may have any suitable length, for example, between 1 and 20 microseconds. The series of bursts includes a gap between bursts of any suitable length, for example, between 100 microseconds to 1000 milliseconds. Each burst may include any suitable number of pulses, for example, up to 100 pulses. The series of bursts may include any suitable number of bursts, for example, up to 100 bursts. The various parameters of the electrical signals (such as amplitude, pulse length, gap, and number of pulses and bursts) may be suitably adjusted to provide an electrical field which causes IRE and a lesion in the tissue to a depth of at least 2-3 mm without substantially heating surrounding tissue.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and subcombinations of the various features described hereinabove, as well as variations and modifications thereof which would occur to persons skilled in the art upon reading the foregoing description and which are not disclosed in the prior art.

### ASPECTS OF THE INVENTION

1. An electroporation method, comprising:
   inserting a catheter having multiple electrodes into a chamber of a heart;
   applying an electrical field using at least two of the electrodes to tissue of the chamber of the heart at a given location within the chamber with an amplitude sufficient to cause reversible electroporation, but below a threshold for irreversible electroporation; and
   measuring an effect of the reversible electroporation on electrical activation signals in the tissue of the chamber of the heart in a vicinity of the location.
2. The method according to aspect 1, wherein the electrical field is less than 450 Volts per centimeter.
3. The method according to aspect 2, further comprising generating a pulsed electrical signal and wherein the applying the electrical field includes applying the electrical field using the at least two electrodes responsively to the generated pulsed electrical signal.
4. The method according to aspect 3, wherein the pulsed electrical signal includes a series of biphasic pulses, each biphasic pulse including a positive and a negative phase pulse.
5. The method according to aspect 3, wherein the pulsed electrical signal includes a series of bursts, each burst including a series of pulses.
6. The method according to aspect 5, wherein: each of the pulses has a pulse length between 1 and 20 microseconds; and the series of bursts includes a gap between bursts of between 100 microseconds to 1000 milliseconds.
7. The method according to aspect 6, wherein: each burst includes up to 100 of the pulses; and the series of bursts includes up to 100 bursts.
8. The method according to aspect 1, further comprising:
   rendering to a display an indication of the electrical activation signals in the tissue of the chamber of the heart in the vicinity of the location; and then
   applying another electrical field using at least two of the electrodes to the tissue of the chamber of the heart at the given location within the chamber with an amplitude sufficient to cause irreversible electroporation.
9. The method according to aspect 8, further comprising:
   generating an electroanatomic map of the chamber of the heart responsively to the electrical activation signals; and
   rendering the electroanatomic map to the display.
10. The method according to aspect 8, wherein the electric field with the amplitude sufficient to cause reversible electroporation but below a threshold for irreversible electroporation is less than 450 Volts per centimeter, and the other electric field with the amplitude sufficient to cause irreversible electroporation is greater than 800 Volts per centimeter.
11. The method according to aspect 10, further comprising generating a pulsed electrical signal and wherein the applying the other electrical field includes applying the other electrical field using the at least two electrodes responsively to the generated pulsed electrical signal.
12. The method according to aspect 11, wherein the pulsed electrical signal includes a series of biphasic pulses, each biphasic pulse including a positive and a negative phase pulse.
13. The method according to aspect 11, wherein the pulsed electrical signal includes a series of bursts, each burst including a series of pulses.
14. The method according to aspect 13, wherein: each of the pulses has a pulse length between 1 and 20 microseconds; and the series of bursts includes a gap between bursts of between 100 microseconds to 1000 milliseconds.
15. The method according to aspect 14, wherein: each burst includes up to 100 of the pulses; and the series of bursts includes up to 100 bursts.

## Claims

1. An electroporation system, comprising:
a catheter including multiple electrodes, and configured to be inserted into a chamber of a heart;
a signal generator coupled to at least two of the electrodes, and configured to generate an electrical signal for supply to the at least two electrodes which responsively to the electrical signal apply an electrical field to tissue of the chamber of the heart at a given location within the chamber, the electrical field having an amplitude sufficient to cause reversible electroporation, but below a threshold for irreversible electroporation; and
processing circuitry configured to: receive from the catheter electrical activation signals in the tissue of the chamber of the heart in a vicinity of the location; and measure an effect of the reversible electroporation on the electrical activation signals in the tissue of the chamber of the heart in a vicinity of the location.

2. The system according to claim 1, wherein the electrical field is less than 450 Volts per centimeter.

3. The system according to claim 2, wherein the electrical signal is a pulsed electrical signal.

4. The system according to claim 2, wherein the pulsed electrical signal includes a series of biphasic pulses, each biphasic pulse including a positive and a negative phase pulse.

5. The system according to claim 2, wherein the pulsed electrical signal includes a series of bursts, each burst including a series of pulses.

6. The system according to claim 5, wherein: each of the pulses has a pulse length between 1 and 20 microseconds; and the series of bursts includes a gap between bursts of between 100 microseconds to 1000 milliseconds.

7. The system according to claim 6, wherein: each burst includes up to 100 of the pulses; and the series of bursts includes up to 100 bursts.

8. The system according to claim 1, wherein:
the processing circuitry is configured to render to a display an indication of the electrical activation signals in the tissue of the chamber of the heart in the vicinity of the location; and
the signal generator is configured to generate another electrical signal for supply to at least two of the electrodes which responsively to the other electrical signal apply another electrical field to tissue of the chamber of the heart at the location within the chamber with an amplitude sufficient to cause irreversible electroporation.

9. The system according to claim 8, wherein the processing circuitry is configured to:
generate an electroanatomic map of the chamber of the heart responsively to the electrical activation signals; and
render the electroanatomic map to the display.

10. The system according to claim 8, wherein the electric field with the amplitude sufficient to cause reversible electroporation but below a threshold for irreversible electroporation is less than 450 Volts per centimeter, and the other electric field with the amplitude sufficient to cause irreversible electroporation is greater than 800 Volts per centimeter.

11. The system according to claim 10, wherein the other electrical signal is a pulsed electrical signal.

12. The system according to claim 11, wherein the pulsed electrical signal includes a series of biphasic pulses, each biphasic pulse including a positive and a negative phase pulse.

13. The system according to claim 11, wherein the pulsed electrical signal includes a series of bursts, each burst including a series of pulses.

14. The system according to claim 13, wherein: each of the pulses has a pulse length between 1 and 20 microseconds; and the series of bursts includes a gap between bursts of between 100 microseconds to 1000 milliseconds.

15. The system according to claim 14, wherein: each burst includes up to 100 of the pulses; and the series of bursts includes up to 100 bursts.
